(19) Europäisches Patentamt
European Patent Office
Office européen des brevets

(11) **EP 4 474 818 A1**

(12) **EUROPEAN PATENT APPLICATION**

(43) Date of publication:
**11.12.2024 Bulletin 2024/50**

(51) International Patent Classification (IPC):
**G01N 33/28** $^{(2006.01)}$ **G01N 27/22** $^{(2006.01)}$

(21) Application number: **24177701.0**

(22) Date of filing: **23.05.2024**

(52) Cooperative Patent Classification (CPC):
**G01N 33/2858; G01N 27/22;** F16N 29/04;
F16N 2200/04; F16N 2210/02; F16N 2210/08;
G01N 27/221; G01N 27/226; G01N 27/227;
G01N 2015/0053

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **24.05.2023 US 202318201439**

(71) Applicant: **PRATT & WHITNEY CANADA CORP.**
**Longueuil, Québec J4G 1A1 (CA)**

(72) Inventors:
• **KHAN, Sarmad**
**(01BE5) Longueuil, J4G 1A1 (CA)**
• **ARNONE, Daniel**
**(01BE5) Longueuil, J4G 1A1 (CA)**

(74) Representative: **Dehns**
**10 Old Bailey**
**London EC4M 7NG (GB)**

(54) **CAPACITIVE CHIP DETECTOR**

(57)    Capacitive chip detectors (28; 328) for detecting metallic chips (42A, 42B, 42C) in engine fluid (26) of an engine (10) are provided. A capacitive chip detector (28; 328) includes a capacitor having first and second electric conductors (36, 38). The detector (28; 328) further includes an inlet (48) for receiving engine fluid (26) between the first and second electric conductors (36, 38). The capacitive chip detector (28; 328) includes an electric circuit (34; 334) including the capacitor, which capacitance changes when metallic chips (42A, 42B, 42C) are present between the first and second electric conductors (36, 38). The electric circuit (34; 334) provides an output indicative of a chip detection between the first and second electric conductors (36, 38).

FIG. 2A

EP 4 474 818 A1

## Description

TECHNICAL FIELD

The disclosure relates generally to health monitoring of engines, and more particularly to detecting chips in fluids of aircraft engines.

BACKGROUND

A magnetic chip detector is commonly found in a lubrication system of an aircraft engine to detect the presence of metallic chips in the lubricating fluid. The chip detector is immersed in the lubricating fluid so as to be exposed to the chips carried by the lubricating fluid. The presence of chips in the lubricating fluid may indicate a developing and/or impending mechanical problem exhibiting excessive wear of one or more components of the aircraft engine interacting with the lubrication system. The presence of metal chips in engine fluid can be indicative of a deteriorating engine health condition and it is desirable to improve chip detection in aircraft engines.

SUMMARY

In one aspect, the present invention describes a capacitive chip detector for detecting chips in engine fluid of an engine. The capacitive chip detector comprises:
a capacitor including:

a first electric conductor having a first polarity;

a second electric conductor having a second polarity opposite the first polarity, the first electric conductor being spaced apart from the second electric conductor; and

an electric field between the first electric conductor and the second electric conductor;

an inlet for receiving the engine fluid between the first electric conductor and the second electric conductor; and

an electric circuit including the capacitor, the electric circuit providing an output indicative of a change in capacitance of the capacitor caused by a presence of one or more chips between the first electric conductor and the second electric conductor.

In an embodiment of the above, the capacitive chip detector further comprises a strainer for collecting the one or more chips between the first electric conductor and the second electric conductor.
In an embodiment according to any of the previous embodiments, the strainer comprises a mesh sized to prevent passage of chips exceeding a threshold chip size.

In an embodiment according to any of the previous embodiments, the electric circuit provides the output based on a magnitude of a potential difference across the capacitor.
In an embodiment according to any of the previous embodiments, the electric circuit provides the output when the magnitude of the potential difference across the capacitor differs from a reference potential difference magnitude by more than a threshold amount.
In an embodiment according to any of the previous embodiments, the electric circuit provides the output when the magnitude of the potential difference across the capacitor is greater than a reference potential difference magnitude by more than a threshold amount.
In an embodiment according to any of the previous embodiments, the capacitor is a cylindrical capacitor where the first electric conductor is an inner cylinder and the second electric conductor is an outer cylinder surrounding the inner cylinder.
In an embodiment according to any of the previous embodiments, the capacitive chip detector comprises an outlet downstream of the capacitor for letting the engine fluid out of the capacitive chip detector and a strainer disposed between the inlet and the outlet for collecting the one or more chips between the first electric conductor and the second electric conductor.
In another aspect, the present invention describes an aircraft engine comprising:

a lubrication system for distributing lubricating fluid to one or more lubrication loads; and

a capacitive chip detector for detecting one or more chips carried by the lubricating fluid, the capacitive chip detector including:
a cylindrical capacitor including:

an electrically conductive inner cylinder having a first polarity;

an electrically conductive outer cylinder having a second polarity opposite the first polarity, the outer cylinder surrounding the inner cylinder and being radially spaced apart from the inner cylinder; and

an electric field between the inner cylinder and the outer cylinder;

an inlet for receiving the lubricating fluid between the inner cylinder and the outer cylinder; and

an electric circuit including the cylindrical capacitor, the electric circuit providing an output indicative of a change in capacitance of the cylindrical capacitor caused by an arrival of one or more chips between the inner cylinder and the outer cylinder.

In an embodiment of the above, the capacitive chip detector includes a strainer disposed downstream of the inlet for preventing the one or more chips from exiting the capacitive chip detector.

In an embodiment according to any of the previous embodiments, the strainer includes a mesh sized to prevent passage of chips exceeding a threshold chip size.

In an embodiment according to any of the previous embodiments, the strainer comprises one or more apertures sized to permit passage of the lubricating fluid and to prevent passage of chips exceeding a threshold chip size.

In an embodiment according to any of the previous embodiments, the output includes a magnitude of a potential difference across the capacitor.

In another aspect, the present invention describes a method of detecting metallic chips in engine fluid of an aircraft engine using a capacitive chip detector including a capacitor defined by a first electric conductor having a first polarity and a second electric conductor having a second polarity opposite the first polarity, the first electric conductor being spaced apart from the second electric conductor, the method comprising:

receiving the engine fluid in an electric field between the first electric conductor and the second electric conductor; and

using an electric circuit including the capacitor, generating an output indicative of a change in capacitance of the capacitor caused by a presence of one or more chips between the first electric conductor and the second electric conductor.

In an embodiment of the above, the method comprises preventing passage of the one or more chips out of an outlet of the capacitive chip detector.

In an embodiment according to any of the previous embodiments, the method comprises passing the engine fluid through a strainer within the capacitive chip detector.

In an embodiment according to any of the previous embodiments, the strainer includes one or more apertures sized to prevent passage of chips exceeding a threshold size.

In an embodiment according to any of the previous embodiments, the method comprises removing the strainer from the capacitive chip detector to access the one or more chips.

In an embodiment according to any of the previous embodiments, the capacitor has a cylindrical configuration.

In an embodiment according to any of the previous embodiments, generating the output based on a magnitude of a potential difference differs from a reference potential difference magnitude by more than a threshold amount.

Further details of these and other aspects of the subject matter of this application will be apparent from the detailed description included below and the drawings.

DESCRIPTION OF THE DRAWINGS

Reference is now made to the accompanying drawings, in which:

FIG. 1 is a schematic axial cross-section view of a turbofan gas turbine engine including a capacitive chip detector as described herein;

FIG. 2A is a schematic representation of an example capacitive chip detector of the engine of FIG. 1;

FIG. 2B is a schematic representation of an electric circuit associated with the capacitive chip detector of FIG. 2A;

FIG. 3A is a schematic representation of an example capacitive chip detector of the engine of FIG. 1, in accordance with some embodiments;

FIG. 3B is a schematic representation of an electric circuit associated with the capacitive chip detector of FIG. 3A; and

FIG. 4 is a flow diagram of a method of detecting metallic chips in engine fluid of an engine.

DETAILED DESCRIPTION

The present disclosure relates to systems and methods for detecting metallic chips in engine fluids (e.g., coolant, liquid fuel, lubricating fluid such as oil). In some embodiments, the systems and methods described herein may help assess a condition of an engine by using one or more chip detectors and/or collectors to reduce the probability of generating nuisance chip detections and associated annunciations or alarms. For example, the systems and methods described herein may reduce the probability of nuisance chip detections associated with the accumulation of acceptable smaller/fine magnetic debris/particles, commonly referred to as "fuzz" at a chip detector immersed in engine fluid. Some fuzz can be generated during the normal operation of an aircraft engine and may not necessarily be indicative of a developing or impending mechanical problem. For example, such fuzz can normally be generated during the initial period (e.g., a few hundred hours) of operation of an aircraft engine following initial entry into service or following extensive maintenance such as an overhaul. This initial period is also known as the engine's "break-in" period. Chip detections caused by the accumulation of the acceptable and relatively harmless fuzz, during the break-in period for example, oppose the design intent of the chip detector and are undesirable since they do not provide an accurate indication of a possible developing or impending problem.

Metallic chips that are carried by the engine fluid may be from metallic engine parts such as gear teeth or bearings, for example. Some disc type magnetic chip detectors use one disk-shaped magnet placed in a non-conductive and non-magnetically permeable isolator with two conductive steel end caps which, in combination, create a single chip capture zone. When a metallic chip of a sufficient size is attracted to the capture zone by the magnet, the metallic chip bridges (e.g., short-circuits) the gap between the steel end caps to complete an electric circuit that includes the gap. A controller or other annunciation circuitry connected to the magnetic chip detector may detect this change in resistance caused by the metallic chip and indicate to a pilot of the aircraft via a cockpit indication the presence of one or more magnetic chips having been detected by the magnetic chip detector.

Magnetic chip detectors may use rare earth magnets that provide a relatively high capture efficiency. This can result in a high sensitivity of a magnetic chip detector, which may increase the probability of nuisance detections. For example, a magnetic chip detector with a single capture zone may function as an on/off switch and may trigger an annunciation based on a relatively harmless single metallic filament having been captured in the single capture zone. Once the chip detection is triggered by one or more chips, the pilot may be required to abort the mission mid-flight, return to the ground (e.g., base) as soon as possible, and remove (or have maintenance personnel remove) the magnetic chip detector for visual inspection. The visual inspection verifies whether significant debris has been collected by the magnetic chip detector and hence whether maintenance is required before the aircraft can be dispatched again. If the detection is triggered by an insignificant metallic filament or other insignificant metallic particle(s), such detection can be a nuisance to the pilot and/or aircraft operator.

In some embodiments, the chip detectors described herein may reduce the probability of single chip detections and other nuisance detection events. In some embodiments, the chip detectors described herein may reduce the probability of fuzz-induced nuisance detections. In some embodiments, the chip detectors described herein may provide an indication of a degree of contamination that allows a system to discriminate between harmless/tolerable contamination and gross contamination indicative of a developing and/or impending mechanical problem. In some embodiments, the chip detectors described herein may provide an ability to discriminate between small or large chips being detected.

Aspects of various embodiments are described through reference to the drawings. Even though the description below is provided in relation to lubricating fluid, it is understood that some embodiments of the chip detectors, systems and methods described herein may also be used on other types of engine fluids such as engine coolant and liquid fuel for example.

The term "connected" may include both direct connection (in which two elements that are connected to each other contact each other) and indirect connection (in which at least one additional element is located between the two elements).

The term "substantially" as used herein may be applied to modify any quantitative representation which could permissibly vary without resulting in a change in the basic function to which it is related.

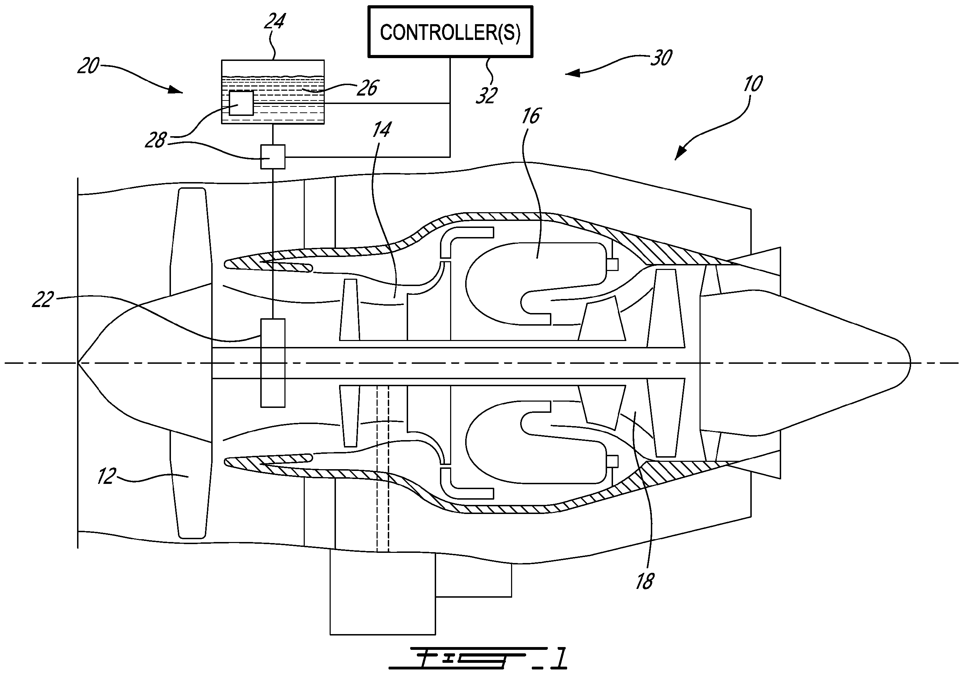

FIG. 1 is a schematic axial cross-section view of aircraft engine 10 (referred hereinafter as "engine 10") of a turbofan gas turbine engine preferably provided for use in subsonic flight, generally comprising, in serial flow communication, fan 12 through which ambient air is propelled, multistage compressor 14 for pressurizing the air, combustor 16 in which the compressed air is mixed with fuel and ignited for generating an annular stream of hot combustion gases, and turbine section 18 for extracting energy from the combustion gases. Engine 10 may be mounted to an aircraft and used to propel such aircraft. Even though FIG. 1 shows engine 10 being of the turbofan type, it is understood that aspects of the present disclosure are also applicable to other (e.g., turboshaft, turboprop, piston) types of aircraft engines.

Engine 10 may include lubrication (and/or other fluid) system 20 shown schematically and partially in FIG. 1. Lubrication system 20 may serve to lubricate, cool and clean one or more lubrication loads 22 such as bearings and gears of engine 10. Lubrication system 20 may include tank 24 and other components such as one or more pumps, one or more valves, and one or more filters. Tank 24 may be a reservoir containing a supply of lubricating fluid 26 such as oil for use by lubrication system 20. Lubrication system 20 may include one or more capacitive chip detectors (CCDs) 28. For example, lubrication system 20 may include a single CCD 28 or a plurality of CCDs 28 disposed at different locations within lubrication system 20. CCD 28 may be at least partially immersed in lubricating fluid 26 during operation. For example, CCD 28 may be disposed inside tank 24, inside a gearbox, or in a scavenge line.

CCD 28 may be part of chip detection system 30 (referred hereinafter as "system 30") and may be associated with and/or may be part of engine 10. System 30 may include controller 32 or other detection circuitry operatively connected to CCD 28. In various embodiments, controller 32 may include or form part of a Full Authority Digital Engine Control (FADEC) which may, for example, include one or more digital computer(s) or other data processors, sometimes referred to as electronic engine controller(s) (EEC) and related accessories that control at least some aspects of performance of engine 10. Controller 32 may, for example, be configured to make decisions regarding the control of engine 10. Controller 32 may include one or more data processors and non-transitory machine-readable memory. Controller 32 may receive input(s) from CCD 28, perform one or more procedures or steps defined by instructions stored in the memory to generate output(s) such as triggering a suitable annunciation to a pilot of the aircraft for example. Various aspects of the present disclosure may be embodied as systems, devices, methods and/or computer program products.

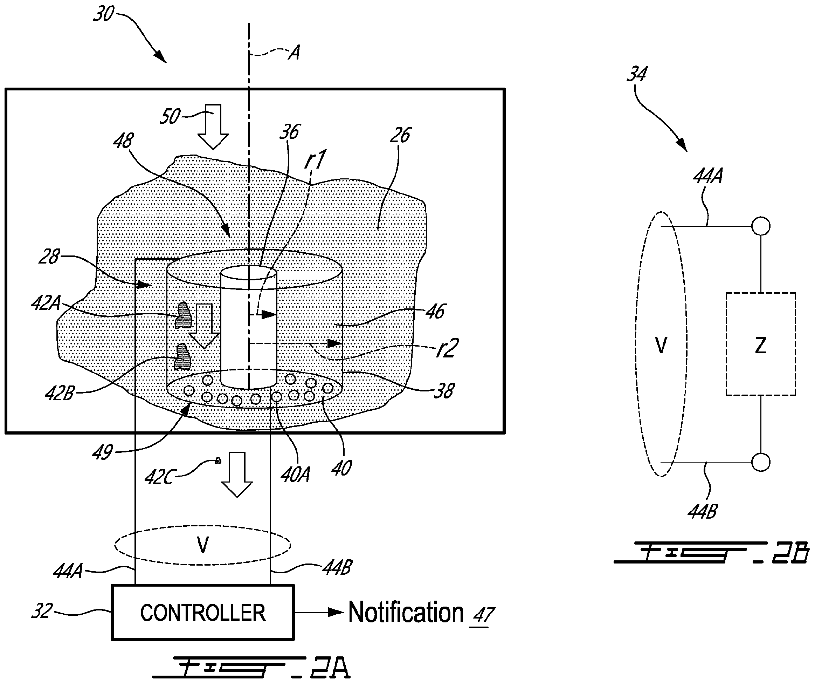

FIG. 2A is an exemplary schematic representation of CCD 28 shown as part of chip detection system 30 of engine 10. In some embodiments, CCD 28 includes a capacitor having first and second electric conductors separated by a dielectric. In some embodiments, the first and second electric conductors may be flat or curved plates that are spaced apart. As depicted in FIG. 2A, the capacitor of CCD 28 may have a cylindrical configuration where the first conductor is an inner cylinder 36 and the second conductor is an outer cylinder 38 surrounding inner cylinder 36. Inner cylinder 36 and outer cylinder 38 may be radially spaced apart to define a fluid-receiving gap (e.g., space 46) therebetween. Inner cylinder may have a radius $r_1$, and outer cylinder may have a radius $r_2$. In some embodiments, inner cylinder 36 and outer cylinder 38 are concentric or coaxial about axis A. In some embodiments, inner cylinder 36 and outer cylinder 38 may have different or opposite polarities, such that a circumferential electric field is created by the separation of charge by a dielectric or non-conductive material or space 46 between inner cylinder 36 and outer cylinder 38. In some embodiments, inner cylinder 36 may be positively charged. In some embodiments, outer cylinder 38 may be grounded. In still other embodiments, outer cylinder 38 may be negatively charged.

Although various embodiments described herein refer to CCD 28, 328 including a cylindrical capacitor having an inner cylinder 36 and outer cylinder 38, it is contemplated that some embodiments may feature other geometric configurations for CCD 28, 328. For example, CCD 28, 328 may have a box-shaped (or cuboid) configuration in which the capacitor is defined by two flat conductive plates (e.g. inner and outer plates) separated by a dielectric.

In some embodiments, CCD 28 includes an inlet 48 for receiving lubricating fluid 26 which is travelling in direction 50 through space 46 between inner cylinder 36 and outer cylinder 38. Although various example embodiments described herein relate to lubricating fluid, it is contemplated that fluids may also include, for example, coolant and liquid fuels. In some embodiments, CCD 28 includes a strainer 40 at outlet 49 of CCD 28 which is downstream from inlet 48. As depicted, in some embodiments, strainer 40 may include one or more apertures 40A for allowing lubricating fluid 26 to exit outlet 49 of CCD 28. In some embodiments, the streamwise direction of system 30 is inlet 48 upstream, outlet 49 downstream of inlet 48, with the capacitor of CCD 28 located between inlet 48 and outlet 49. In some embodiments, a directional (e.g. one-way) valve may be disposed proximal to inlet 48. The directional valve may be configured to prevent the back flow of lubricating fluid 26 in a direction opposite to direction 50 to help retain chips 42A that have been collected in CCD 28.

In some embodiments, apertures 40A may have a uniform shape and/or size. In some embodiments, apertures 40A may have varying shapes and/or sizes. In some embodiments, apertures 40A may be sized so as to allow passage of chips 42C which are smaller than a predetermined threshold particle size (e.g. fuzz), while preventing passage of chips 42A, 42B which are larger than the predetermined threshold size (and therefore may be indicative of deteriorating engine components), thereby causing chips 42A, 42B to accumulate within CCD 28 over time. In some embodiments, strainer 40 may be made from a non-conductive material so as to reduce the likelihood of an electrical connection being formed between the inner cylinder 36 and outer cylinder 38.

Although FIG. 2A depicts strainer 40 as including one or more apertures 40A, it is contemplated that in other embodiments, strainer 40 may include a mesh with a selected mesh size configured to allow passage of lubricating fluid 26 and chips 42C which are smaller than the mesh size (e.g. fuzz), and prevent passage of chips 42A, 42B which are larger than the mesh size.

It will be appreciated that as CCD 28 includes two conductively charged cylinders separated by an insulator, CCD 28 is a cylindrical capacitor. The capacitance, $C$, of a cylindrical capacitor is

$$C = \frac{2\pi\varepsilon L}{\ln(\frac{r_2}{r_1})}$$

, where L is the length of the cylinder, $r_2$ is the radius of the outer cylinder, $r_1$ is the radius of the inner cylinder, and $\varepsilon$ is the permittivity of free space.

As lubricating fluid 26 passes through CCD 28, some chips 42A may be prevented from passing through strainer 40. Over time, such chips 42A, 42B may accumulate within dielectric space 46 of CCD 28, and may be expected not to escape from CCD 28 due to lubricating fluid 26 moving in direction 50 towards strainer 40. When chips 42A, 42B are metallic (or any material which conducts electricity), the accumulation of conductors within dielectric space 46 will affect the permittivity $\varepsilon$ of CCD 28. Given that the length and the radii of inner cylinder 36 and outer cylinder 38 are constants, a change in the permittivity of CCD 28 will result in a change in the capacitance of CCD 28. For example, a build-up of conducting (e.g. metallic) chips would be expected to decrease the capacitance of CCD 28, whereas a build-up of insulating chips (e.g. sand and dust) would be expected to increase the capacitance of CCD 28. In some embodiments, the inclusion of a directional (e.g., one-way) valve disposed upstream of CCD 28 may prevent one or more accumulated conducting chips from escaping the confines of space 46 between inner cylinder 36 and outer cylinder 38.

When an AC current or voltage with a frequency *f* is applied to a capacitor, the reactance of the capacitor (i.e., the resistance to alternating current) is given by $X$ = $\frac{1}{2\pi f C}$ for an ideal capacitor. For an ideal capacitor, the magnitude of the total impedance, $Z$, is given by $|Z| = X$. For a non-ideal capacitor with a non-zero resistance, the total impedance, $Z$, is given by $Z = R + jX$, and the total magnitude of impedance is given by

$$|Z| = \sqrt{R^2 + X^2}.$$

In some embodiments, as metallic chips 42A, 42B accumulate within space 46 of CCD 28, the resulting decrease in the capacitance of CCD 28 will result in a corresponding increase in the reactance of CCD 28, assuming the frequency of the voltage or current applied is kept constant. This property of CCD 28 may be advantageously used to detect the arrival and/or presence of metallic chips 42A, 42B between inner cylinder 36 and outer cylinder 38 of CCD 28.

In some embodiments, CCD 28 may form part of an electric circuit 34 which may include first output terminal 44A and second output terminal 44B together defining a single interface between CCD 28 and controller 32. As shown in FIG. 2B, the total impedance of CCD 28 may be represented as a component with impedance Z. In some embodiments, an alternating current or voltage may be applied across CCD 28 and the voltage across CCD 28 may be measured by, or transmitted to, controller 32. The magnitude of the voltage drop across CCD 28 may serve as a single output for controller 32 to use as an indication of whether or not a chip detection has occurred at CCD 28 and whether a notification 47 is warranted for further investigation and/or inspection and/or repair.

For example, if an AC voltage with a constant frequency is applied to circuit 34, in the absence of metallic chips 42A, 42B within CCD 28, a reference potential difference across CCD 28 may be established. As metallic chips 42A, 42B subsequently begin to accumulate in space 46 due to strainer 40 preventing the movement of chips exceeding a threshold size, the resulting decrease in capacitance of CCD 28 will cause an increase in reactance and total impedance, thereby resulting in an increase in the voltage across CCD 28 relative to the reference potential difference. In some embodiments, a threshold difference for the potential difference across CCD 28 relative to the reference potential difference in the absence of metallic chips 42A, 42B may be defined or otherwise set. When the difference in the voltage drop across CCD 28 exceeds the reference value by more than this threshold amount, controller 32 may be configured to sent an alert or notification 47 to draw attention to the possible need for maintenance or inspection for engine.

Accordingly, a chip detection at CCD 28 may be detected by way of a change in potential difference V across the cylindrical capacitor, which may be determined at controller 32. Upon detection of the change in potential difference V that is indicative of a legitimate chip detection, controller 32 may initiate notification 47 which may alert a pilot of the aircraft and/or another interested party. Notification 47 may be visual (e.g., indicator light or message) or aural (e.g., tone or spoken message).

Advantageously, the likelihood of alerts being triggered when unnecessary (so-called "false alarms") may be reduced given that the mesh size or aperture 40A size for strainer 40 may be selected so as to allow chips which are smaller in size (e.g. fuzz) to pass through strainer 40. Allowing fuzz to pass through strainer 40 may reduce the likelihood of metallic fuzz particles accumulating within space 46 (which may eventually result in a sufficient accumulation of metallic fuzz to result in the capacitance of CCD 28 falling sufficiently to trigger controller 32 to signal an alert).

It may be further appreciated that in some embodiments, CCD 28 need not involve the use of magnets to attract chips 42. In other words, CCD 28 may be devoid of magnets. While other types of detectors may rely on the use of magnets to attract metallic chips 42 to "bridge" gaps between conductors to establish the presence of chips 42, the CCD 28 instead relies on changes in capacitance rather than the establishment of any physical electrical connection by chips 42. As such, CCD 28 may alleviate common problems associated with the use of magnetic chip detectors, such as the oversensitivity of magnets used (resulting in too many chips being attracted, including fuzz), and the inability to both determine the size of particles being captured, as well as the inability to distinguish between the physical size of chips 42 being captured.

In various embodiments disclosed herein, the single potential difference value V may provide a single and informative input to controller 32 and allow controller 32 to determine the state of CCD 28. For example, potential difference value V may be the sole piece of information transferred from CCD 28 to controller 32. Controller 32 may then use potential difference value V with one or more rules available to controller 32 to determine whether notification 47 or other action is warranted. The rules may include actions associated with predetermined values for potential difference value V or predetermined ranges of values for potential difference V. The rules and the mesh size and/or aperture 40A size may be selected to provide a sensitivity suitable for providing legitimate chip detections and notifications 47.

The use of the single input may also facilitate integration of some embodiments of CCD 28 disclosed herein with new or existing aircraft engines. For example, the retrofitting of CCD 28 into engine 10 may require little to no hardware modifications, but may require software modifications to apply rules and carry out desired actions based on potential difference value V.

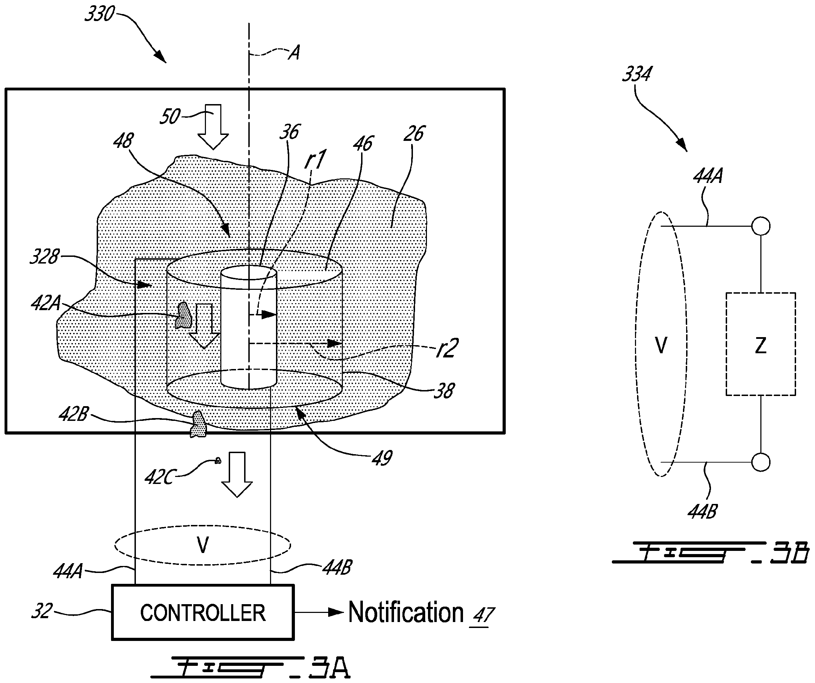

FIG. 3A is a schematic representation of an example CCD 328, in accordance with some embodiments, shown as part of chip detection system 330 of engine 10.

As depicted, CCD 328 is a cylindrical capacitor similar to CCD 28, and includes an inner cylinder 36 and an outer cylinder 38. Inner cylinder 36 may have a radius $r_1$, and outer cylinder 38 may have a radius $r_2$. In some embodiments, inner cylinder 36 and outer cylinder 38 are concentric or coaxial about axis A. In some embodiments, inner cylinder 36 and outer cylinder 38 may have different or opposite polarities, such that a circumferential electric field is created by the separation of charge by a dielectric or non-conductive material or space 46 between inner cylinder 36 and outer cylinder 38. In some embodiments, inner cylinder 36 may be positively charged. In some embodiments, outer cylinder 38 may be grounded. In still other embodiments, outer cylinder 38 may be negatively charged.

In some embodiments, CCD 328 includes an inlet 48 for receiving lubricating fluid 26 which is travelling in direction 50. As depicted, CCD 328 does not include any strainer or collection system at an outlet 49 of CCD 328. In some embodiments, the streamwise direction of system 330 is inlet 48 upstream, outlet 49 downstream of inlet 48, with the capacitor of CCD 328 located between inlet 48 and outlet 49. As such, CCD 328 may allow passage of substantially all chips 42A, 42B, 42C, irrespective of the size of the chips. As such, there is no accumulation of metallic chips in CCD 328 as lubricating fluid 26 flows through CCD 328.

As with CCD 28, CCD 328 includes two conductively charged cylinders separated by an insulator and as such is a cylindrical capacitor. The capacitance, C, of CCD 328 is

$$C = \frac{2\pi\varepsilon L}{\ln(\frac{r_2}{r_1})}$$

, where L is the length of the cylinder, $r_2$ is the radius of the outer cylinder 38, $r_1$ is the radius of inner cylinder 36, and $\varepsilon$ is the permittivity of free space.

In contrast to the operation of CCD 28, as lubricating fluid 26 passes through CCD 328, substantially no chips 42A will be prevented from passing from the inlet 48 to outlet 49 of CCD 328. As such, it is not expected that the capacitance of CCD 328 will decrease over time, as metallic chips will not be accumulating within space 46 (depicted as chip 42B exiting CCD 328 in FIG. 3A). Nevertheless, when chips 42A, 42B, 42C are metallic (or any material which conducts electricity), the permittivity of space 46 in CCD 328 will be affected as chips 42A, 42B, 42C travel from the inlet 48 to outlet 49, resulting in temporary or transient decreases in the capacitance, C, of CCD 328 while the metallic chips are present between the inner cylinder 36 and outer cylinder 38. For example, the temporary presence of a relative large metallic chip 42A in space 46 would be expected to temporarily decrease the capacitance of CCD 328 by a first amount, and the temporary presence of a relatively small metallic chip 42C (e.g. fuzz) would be expected to temporarily decrease the capacitance of CCD 328 by a second amount which is less than the first amount.

In some embodiments, as metallic chips 42A, 42B, 42C pass through space 46 of CCD 328, the resulting decrease in the capacitance of CCD 28 will result in a corresponding temporary increase in the reactance of CCD 28, assuming the frequency of the voltage or current applied is kept constant. This property of CCD 328 may be advantageously used to detect the presence of metallic chips 42A, 42B, 42C between inner cylinder 36 and outer cylinder 38 of CCD 28.

In some embodiments, CCD 328 may form part of an electric circuit 334 which may include first output terminal 44A and second output terminal 44B together defining a single interface between CCD 328 and controller 32. As shown in FIG. 3B, the total impedance of CCD 328 together with chips 42A, 42B, 42C may be represented as a component with impedance Z. In some embodiments, an alternating current or voltage may be applied across CCD 328 and the voltage across CCD 328 may be measured by, or transmitted to, controller 32. The magnitude of the voltage drop across CCD 328 may serve as an output for controller 32 to use as an indication of whether or not a chip detection has occurred at CCD 328 and whether a notification 47 is warranted for further investigation and/or inspection and/or repair.

For example, controller 32 may be operable to store, in memory, data points over time of the capacitance of CCD 328 during operation of engine 10. Such data points may be analyzed for trends in capacitance changes. For example, a data model may be generated using one or more of linear regression, machine learning, neural networks, and the like, to identify subsequent anomalies in the data received at controller 32 based on the observed potential difference (and therefore the changes in capacitance of CCD 328). Spikes (or patterns of data more generally) in the potential difference across CCD 328 may be processed and interpreted as signifying the presence of metallic particles in a fluid line which are of sufficient size to be indicative of a deteriorating engine health condition (or warranting further inspection). Controller 32 may be further configured to interpret aberrations in the potential difference across CCD 328 which are not indicative of a change in capacitance above a threshold amount as being attributable to fuzz and other particles which might not be indicative of deteriorating engine health.

For example, if an AC voltage with a constant frequency is applied to circuit 334, in the absence of metallic chips 42A, 42B within CCD 328, a reference potential difference across CCD 328 may be established. As metallic chips 42A, 42B, 42C subsequently pass through CCD 328 in direction 50, the temporary presence of chips 42A, 42B, 42C may cause a temporary decrease in the capacitance of CCD 328, which will cause a temporary increase in reactance and total impedance, thereby resulting in a temporary increase or spike in the voltage across CCD 328 relative to the reference potential difference value V. In some embodiments, a threshold difference for the potential difference across CCD 328 relative to the reference potential difference in the absence of metallic chips 42A, 42B may be defined or otherwise set. When the difference in the voltage drop across CCD 328 exceeds the reference value by more than this threshold amount, controller 32 may be configured to sent an alert or notification 47 to draw attention to the possible need for maintenance or inspection for engine. In some embodiments, a controller 32 may be configured to detect spikes and anomalies in the potential difference across CCD 328 by comparing the potential difference to a data model based on historical recorded data for engine 10 (this data may be obtained from the engine 10 being monitored, and/or may be based on data obtained from other engines which are the same make and model as engine 10).

Accordingly, a chip detection at CCD 328 may be detected by way of a change in potential difference V across the cylindrical capacitor, which may be determined at controller 32 using a classifier or machine learning model configured to predict the presence of chips. Upon detection of the change in potential difference V that is determined to be indicative of a legitimate chip detection, controller 32 may initiate notification 47 which may alert a pilot of the aircraft and/or another interested party. Notification 47 may be visual (e.g., indicator light or message) or aural (e.g., tone or spoken message).

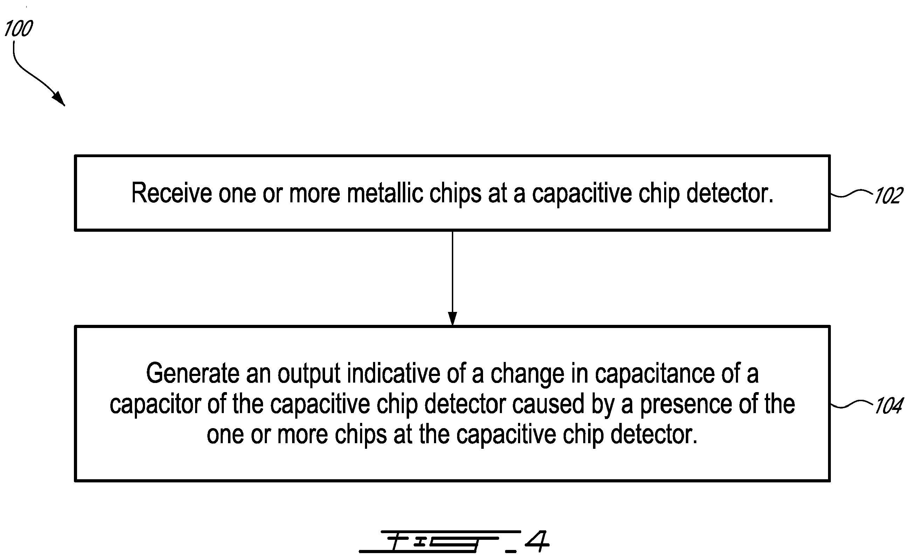

FIG. 4 is a flow diagram of method 100 of detecting metallic chips in engine fluid of an engine. Method 100 may be performed using, for example, CCD 28 described herein or using another CCD. Aspects of method 100 may be combined with other methods or steps described herein. Method 100 may also include aspects of system 30 and of CCD 28. In various embodiments, method 100 may include: receiving the engine fluid in the electric field between the first electric conductor (e.g., inner cylinder 36) and the second electric conductor (e.g., outer cylinder 38) (block 102); and using electric circuit 34 including the capacitor of the CCD 28, generating an output indicative of a change in capacitance of the capacitor caused by the arrival or presence of one or more chips between the first electric conductor and the second electric conductor (block 104).

In some embodiments of method 100, the output may be indicative of the capacitance of the CCD 28 having decreased sufficiently to be indicative of or associated with chip detection.

The embodiments described in this document provide non-limiting examples of possible implementations of the present technology. Upon review of the present disclosure, a person of ordinary skill in the art will recognize that changes may be made to the embodiments described herein without departing from the scope of the present technology.

**Claims**

1. A capacitive chip detector (28; 328) for detecting chips (42A, 42B, 42C) in engine fluid (26) of an engine (10), the capacitive chip detector (28; 328) comprising:

a capacitor including:

a first electric conductor (36) having a first polarity;
a second electric conductor (38) having a second polarity opposite the first polarity, the first electric conductor (36) being spaced apart from the second electric conductor (38); and
an electric field between the first electric conductor (36) and the second electric conductor (38);

an inlet (48) for receiving the engine fluid (26) between the first electric conductor (36) and the second electric conductor (38); and
an electric circuit (34; 334) including the capacitor, the electric circuit (34; 334) providing an output indicative of a change in capacitance of the capacitor caused by a presence of one or more chips (42A, 42B, 42C) between the first electric conductor (36) and the second electric conductor (38).

2. The capacitive chip detector (28) of claim 1, further comprising a strainer (40) for collecting the one or more chips (42A, 42B) between the first electric conductor (36) and the second electric conductor (38), optionally wherein the strainer (40) comprises a mesh sized to prevent passage of chips (42A, 42B) exceeding a threshold chip size.

3. The capacitive chip detector (28) of claim 1, comprising:

an outlet (49) downstream of the capacitor for letting the engine fluid (26) out of the capacitive chip detector (28); and
a strainer (40) disposed between the inlet (48) and the outlet (49) for collecting the one or more chips (42A, 42B) between the first electric conductor (36) and the second electric conductor (38).

4. The capacitive chip detector (28; 328) of any preceding claim, wherein the electric circuit (34; 334) provides the output based on a magnitude of a potential difference across the capacitor.

5. The capacitive chip detector (28; 328) of claim 4, wherein the electric circuit (34; 334) provides the output when the magnitude of the potential difference across the capacitor differs from a reference potential difference magnitude by more than a threshold amount.

6. The capacitive chip detector (28; 328) of claim 5, wherein the electric circuit (34; 334) provides the output when the magnitude of the potential difference across the capacitor is greater than a reference potential difference magnitude by more than a threshold amount.

7. The capacitive chip detector (28; 328) of any preceding claim, wherein the capacitor is a cylindrical capacitor where the first electric conductor (36) is an inner cylinder (36) and the second electric conductor (38) is an outer cylinder (38) surrounding the inner cylinder (36).

8. An aircraft engine (10) comprising:

   a lubrication system (20) for distributing lubricating fluid (26) to one or more lubrication loads (22); and
   a capacitive chip detector (28; 328) for detecting one or more chips (42A, 42B, 42C) carried by the lubricating fluid (26), the capacitive chip detector (28; 328) including:

   a cylindrical capacitor including:

   an electrically conductive inner cylinder (36) having a first polarity;
   an electrically conductive outer cylinder (38) having a second polarity opposite the first polarity, the outer cylinder (38) surrounding the inner cylinder (36) and being radially spaced apart from the inner cylinder (36); and
   an electric field between the inner cylinder (36) and the outer cylinder (38);

   an inlet (48) for receiving the lubricating fluid (26) between the inner cylinder (36) and the outer cylinder (38); and
   an electric circuit (34; 334) including the cylindrical capacitor, the electric circuit (34; 334) providing an output indicative of a change in capacitance of the cylindrical capacitor caused by an arrival of one or more chips (42A, 42B, 42C) between the inner cylinder (36) and the outer cylinder (38), optionally wherein the output includes a magnitude of a potential difference across the capacitor.

9. The aircraft engine (10) of claim 8, wherein the capacitive chip detector (28) includes a strainer (40) disposed downstream of the inlet (48) for preventing the one or more chips (42A, 42B) from exiting the capacitive chip detector (28), optionally wherein:

the strainer (40) includes a mesh sized to prevent passage of chips (42A, 42B) exceeding a threshold chip size; and/or
the strainer (40) comprises one or more apertures (40A) sized to permit passage of the lubricating fluid (26) and to prevent passage of chips (42A, 42B) exceeding a or the threshold chip size.

10. A method of detecting metallic chips (42A, 42B, 42C) in engine fluid (26) of an aircraft engine (10) using a capacitive chip detector (28; 328) including a capacitor defined by a first electric conductor (36) having a first polarity and a second electric conductor (38) having a second polarity opposite the first polarity, the first electric conductor (36) being spaced apart from the second electric conductor (38), the method comprising:

   receiving the engine fluid (26) in an electric field between the first electric conductor (36) and the second electric conductor (38); and
   using an electric circuit (34; 334) including the capacitor, generating an output indicative of a change in capacitance of the capacitor caused by a presence of one or more chips (42A, 42B, 42C) between the first electric conductor (36) and the second electric conductor (38).

11. The method of claim 10, comprising preventing passage of the one or more chips (42A, 42B) out of an outlet (49) of the capacitive chip detector (28).

12. The method of claim 11, comprising passing the engine fluid (26) through a strainer (40) within the capacitive chip detector (28).

13. The method of claim 12, wherein the strainer (40) includes one or more apertures (40A) sized to prevent passage of chips (42A, 42B) exceeding a threshold size, and/or the method comprising removing the strainer (40) from the capacitive chip detector (28) to access the one or more chips (42A, 42B).

14. The method of any of claims 10 to 13, wherein the capacitor has a cylindrical configuration.

15. The method of any of claims 10 to 14, further comprising generating the output based on a magnitude of a potential difference differing from a reference potential difference magnitude by more than a threshold amount.

FIG-1

CONTROLLER → Notification _47_

FIG. 2A

FIG. 2B

EP 4 474 818 A1

CONTROLLER

Notification _47_

FIG.3A

FIG.3B

```
┌─────────────────────────────────────────────────────────────────┐
│                                                                   │
│   Receive one or more metallic chips at a capacitive chip         │   ~102
│   detector.                                                       │
│                                                                   │
└─────────────────────────────────────────────────────────────────┘
                                │
                                ▼
┌─────────────────────────────────────────────────────────────────┐
│                                                                   │
│   Generate an output indicative of a change in capacitance of a   │
│   capacitor of the capacitive chip detector caused by a presence  │   ~104
│   of the one or more chips at the capacitive chip detector.       │
│                                                                   │
└─────────────────────────────────────────────────────────────────┘
```

*100*

_FIG_ 4

**EUROPEAN SEARCH REPORT**

Application Number

EP 24 17 7701

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 8 522 604 B2 (ZHE JIANG [US]; DU LI [US] ET AL.) 3 September 2013 (2013-09-03) | 1-6, 10-13,15 | INV. G01N33/28 |
| Y | * column 4, line 42 - line 46 * <br> * column 5, line 18 - line 25 * <br> * column 5, line 55 - line 62 * <br> * column 5, line 67 - column 6, line 5 * <br> * column 8, line 35 - line 37 * <br> * figures 1,3 * <br> * claims 19,26 * | 7,9,14 | G01N27/22 |
| X | WANG YISHOU ET AL: "In-situ capacitive sensor for monitoring debris of lubricant oil", <br> INDUSTRIAL LUBRICATION AND TRIBOLOGY, <br> vol. 70, no. 7, <br> 10 August 2018 (2018-08-10), pages 1310-1319, XP093219099, <br> GB <br> ISSN: 0036-8792, DOI: <br> 10.1108/ILT-09-2017-0256 | 8 | |
| Y | * page 1311 - page 1313 * <br> * page 1315 * <br> * figures 2(a), 2(b), 4, 11 * | 7,9,14 | **TECHNICAL FIELDS SEARCHED (IPC)** <br> G01N <br> F16N |
| A | US 2015/075268 A1 (QI BAOHUA [US]) 19 March 2015 (2015-03-19) <br> * paragraph [0032] * | 1-15 | |
| A | US 7 129 715 B2 (NGK SPARK PLUG CO [JP]) 31 October 2006 (2006-10-31) <br> * the whole document * | 1-15 | |
| A | US 10 197 488 B2 (PRATT & WHITNEY CANADA [CA]) 5 February 2019 (2019-02-05) <br> * the whole document * | 1-15 | |

-/--

The present search report has been drawn up for all claims

| Place of search | Date of completion of the search | Examiner |
|---|---|---|
| The Hague | 30 October 2024 | Joyce, David |

EPO FORM 1503 03.82 (P04C01)

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DE 100 00 148 A1 (IAV GMBH [DE]; KURT SCHWABE INST FUER MESS UN [DE]) 12 July 2001 (2001-07-12) * the whole document * ----- | 1-15 | |

**TECHNICAL FIELDS SEARCHED (IPC)**

The present search report has been drawn up for all claims

| Place of search | Date of completion of the search | Examiner |
|---|---|---|
| The Hague | 30 October 2024 | Joyce, David |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 24 17 7701

This annex lists the patent family members relating to the patent documents cited in the above-mentioned European search report.
The members are as contained in the European Patent Office EDP file on
The European Patent Office is in no way liable for these particulars which are merely given for the purpose of information.

30-10-2024

| Patent document cited in search report | | Publication date | Patent family member(s) | | Publication date |
|---|---|---|---|---|---|
| US 8522604 | B2 | 03-09-2013 | NONE | | |
| US 2015075268 | A1 | 19-03-2015 | NONE | | |
| US 7129715 | B2 | 31-10-2006 | EP | 1439386 A1 | 21-07-2004 |
| | | | JP | 3840472 B2 | 01-11-2006 |
| | | | JP WO2003029802 A1 | | 20-01-2005 |
| | | | US | 2004263187 A1 | 30-12-2004 |
| | | | WO | 03029802 A1 | 10-04-2003 |
| US 10197488 | B2 | 05-02-2019 | CA | 3004006 A1 | 15-12-2018 |
| | | | US | 2018364141 A1 | 20-12-2018 |
| DE 10000148 | A1 | 12-07-2001 | NONE | | |

EPO FORM P0459

For more details about this annex : see Official Journal of the European Patent Office, No. 12/82